# EUROPÄISCHE PATENTANMELDUNG

(11) **EP 1 376 443 A1**
(43) Veröffentlichungstag der Anmeldung: **02.01.2004**
(21) Anmeldenummer: 02013611.5
(22) Anmeldetag: 19.06.2002
(51) Int. Cl.: G06F 19/00

(54) **Verfahren und Vorrichtung zur Bestimmung von Migrationswegen von Zellen**

(71) Anmelder: BrainLAB AG, 85551 Kirchheim/Heimstetten (DE)
(72) Erfinder: Pedain, Christoph, 81667 München (DE); Hartlep, Andreas, Dr., 80803 München (DE)
(74) Vertreter: Schwabe - Sandmair - Marx

(57) **Zusammenfassung**

Die Erfindung bezieht sich auf ein Verfahren zum Bestimmen von Migrationswegen von Zellen, wobei Daten eines Körpers durch ein bildgebendes Verfahren erfasst werden, aus den erfassten Daten für Migrationswege relevante anatomische Informationen gewonnen werden, und für den Körper Migrationswege berechnet werden sowie auf eine Vorrichtung zur Bestimmung von Migrationswegen von Zellen mit einer Vorrichtung (3) zur Durchführung eines bildgebenden Verfahrens und einer Auswerteeinheit (1) zur individuellen Ermittlung von Migrationswegen in einem Körper basierend auf den von der Vorrichtung (3) zur Verfügung gestellten Daten.

## Beschreibung

Die vorliegende Erfindung bezieht sich auf ein Verfahren und eine Vorrichtung zur Bestimmung oder Modellierung von Migrationswegen von Zellen, insbesondere von Tumorzellen in Gewebe.

Aus der US 6,284,219 B1 sind analytische In-Vivo-Verfahren bekannt, welche die Diagnose von Erkrankungen ermöglichen, bei welchen diskrete biochemische Übertragungswege beteiligt sind. Dabei wird ein bestimmtes Enzym verabreicht und ein bestimmtes Produkt der Wirkung des Enzyms auf einen Körper wird gemessen, wobei das Auftreten und die Konzentration des Produkts mit einer zu untersuchenden Erkrankung im Zusammenhang stehen. Die Rate der Umwandlung des Enzyms in das Produkt wird bestimmt und basierend darauf wird eine Analyse durchgeführt. Somit soll durch die In-Vivo-Bestimmung einer metabolischen Funktion die Durchführung einer Behandlung verbessert werden.

Bekannte biologische und chemische Verfahren können zwar Informationen über bereits vorhandene Erkrankungen liefern, jedoch ist es mit diesen Verfahren nicht möglich Migrationswege von Zellen individuell zu bestimmen oder vorherzusagen.

Es ist bekannt, dass Tumore, insbesondere Glioplastome, sehr wahrscheinlich Metastasen verursachen. Eine einzelne Tumorzelle oder eine kleine Gruppe von Zellen teilt sich beispielsweise von einem bestehenden Tumor ab und wandert in das umliegende Gewebe oder Blutbahnen. Aus diesen Zellen können neue Tumore an anderen Orten entstehen, selbst wenn der primäre Tumor behandelt wurde, zum Beispiel durch Resektion bzw. Herausschneiden, Bestrahlung, lokale Chemotherapie oder andere Behandlungen. Die Wanderung oder Migration von Tumorzellen ist patientenspezifisch und von Individuum zu Individuum aufgrund unterschiedlicher anatomischer Gegebenheiten, verschiedenartiger Ausbildung der Tumore, etc. verschieden und kann durch bekannte Verfahren nicht direkt und präzise vorhergesagt werden. Jedoch würde die Kenntnis der Migrationswege dieser Zellen das frühzeitige Erkennen und Behandeln von möglicherweise entstehenden oder bereits entstandenen kleinen Metastasen ermöglichen, welche häufig durch die bekannten Verfahren erst ab einer gewissen Größe aufgespürt werden können. Da die Behandlung von Metastasen im Frühstadium relativ leicht und mit hohen Erfolgsaussichten durchgeführt werden kann und die Behandlung kleiner Metastasen meist keine größeren Nebenwirkungen hat, könnten durch die möglichst genaue Kenntnis und Vorhersage der Migrationswege von Zellen neue und besonders erfolgversprechende Behandlungen durchgeführt werden.

Es ist eine Aufgabe der vorliegenden Erfindung ein Verfahren und eine Vorrichtung zur Bestimmung von Migrationswegen von Zellen vorzuschlagen, mit welchen Migrationswege individuell bestimmt oder vorhergesagt werden können.

Diese Aufgabe wird durch das Verfahren und die Vorrichtung gemäß den unabhängigen Ansprüchen gelöst. Vorteilhafte Ausführungsformen ergeben sich aus den Unteransprüchen.

Nach dem erfindungsgemäßen Verfahren zur Bestimmung von Migrationswegen von Zellen, wie zum Beispiel Tumorzellen, oder auch durch eine Infusion oder Injektion in einen Körper oder Organismus eingebrachte Zellen, werden individuell für einen Körper charakteristische spezifische oder anatomische Körperdaten, wie zum Beispiel Daten betreffend die Form eines Tumors, die Struktur und Art eines Gewebes, Daten bezüglich des Verlaufs von Blutbahnen, die Form und Lage von Hohlräumen, Zysten, Ödemen oder weitere für einen Körper, eine Körperstruktur oder Gewebe charakteristische Daten erfasst, welche auch zur Ermittlung von Migrationswegen relevant sein können. Die Erfassung dieser Daten erfolgt bevorzugt durch bildgebende Verfahren, wie zum Beispiel Kernspinresonanz(MRI)-Verfahren, Ultraschall-Verfahren, Computertomographie(CT)-Verfahren, Röntgen-, SPECT(Single-Photon-Emission-Computed-Tomography)-Verfahren, wie zum Beispiel PET, oder andere Verfahren. Optional können auch andere oder weitere Untersuchungs- oder Messverfahren verwendet werden, um Daten des Körpers, der Körperstruktur- oder Gewebeeigenschaften zu erhalten, wie zum Beispiel Daten, welche durch eine Biopsie gewonnen wurden. Aus den so gewonnenen für einen Körper, wie zum Beispiel eine Person, spezifischen Daten werden zur Bestimmung von Migrationswegen von Zellen ausgehend von einem bestimmten Bereich oder Punkt, zum Beispiel einem Tumor, spezifische anatomische Informationen ermittelt, wobei hierzu beispielsweise mathematische Modelle und Erkenntnisse, welche aus anderen Untersuchungen gewonnen wurden, wie beispielsweise in der oben erwähnten US 6,284,219 B1 beschrieben, herangezogen werden können. So kann beispielsweise allgemein untersucht werden wie sich eine bestimmte Zellenart in einer bestimmten Art eines Gewebes oder einer Blutbahn bewegt. Aus den so gewonnenen spezifischen anatomischen Informationen können die für einen bestimmten Tumor möglichen spezifischen Migrationswege berechnet werden, wobei beispielsweise auch Wahrscheinlichkeiten dafür angegeben werden können, dass sich Zellen von beispielsweise einem bestimmten Tumor oder Gewebebereich über einen bestimmten der so ermittelten Migrationswege ausbreiten und andere Bereiche des Körpers oder Gewebe gefährden, so dass geeignete Gegenmaßnahmen zur Verhinderung der Migration und/oder eine präventive Behandlung von möglichen Metastasen frühzeitig eingeleitet werden können.

Allgemein können auch nicht-bildgebende Verfahren zur Gewinnung von Körperdaten, verwendet werden, wenn mit diesen Daten mögliche Migrationswege berechnet werden können. Ebenso ist es erfindungsgemäß möglich nicht nur Migrationswege von Zellen, sondern auch Migrationswege von anderen Substanzen in einem Körper oder Gewebe zu ermitteln.

Vorteilhaft können aus den Messungen erfindungsgemäß auch Daten gewonnen werden, welche sich auf die Transportmechanismen in einem Körper oder Gewebe beziehen. Solche bekannten Verfahren werden häufig auch als Diffusionsbildgebung bezeichnet. Weiterhin ist es auch möglich bekannte Verfahren zur Perfusionsbildgebung zu verwenden, mit welchen Informationen erhalten werden können, wie bestimmte Stoffe oder Zellen in einem Blutgefäß oder Blutgefäßsystem transportiert werden. Mit Verfahren zur Perfusionsbildgebung können auch Daten zum Stoff- oder Zellentransport an der für eine Vorhersage von Migrationswegen häufig wichtigen Blut-Gehirn-Schranke gewonnen werden, um zum Beispiel bei der Untersuchung und Behandlung von Gehirntumoren mögliche Migrationswege von Stoffen oder Zellen zu untersuchen und ergänzend auch Informationen zur Verfügung zu haben, wie und wo bestimmte Stoffe oder Medikamente eingesetzt, also zum Beispiel injiziert und/oder dosiert werden sollen, um zum Beispiel einen Gehirntumor zu behandeln.

Kombinationen von oben erwähnten Messungen oder auch einzelne Messungen können dafür verwendet werden beispielsweise die Durchlässigkeit von Gewebe in den verschiedenen Raumrichtungen zu bestimmen oder zwischen anatomischen Merkmalen, beispielsweise white matter und grey matter zu unterscheiden.

Die so gewonnenen individuellen anatomischen Informationen und Daten können sowohl zur erfindungsgemäßen Ermittlung oder Berechnung von Migrationswegen von Zellen verwendet werden. Weiterhin ist es auch möglich diese Daten und optional weitere Daten betreffend zum Beispiel die Perfusion oder die Diffusion auch zur Planung, Vorbereitung und/oder Durchführung einer Behandlung eines bestimmten gefährdeten Bereiches des Körpers zu verwenden. Beispielsweise können Daten zur Planung, Vorbereitung oder Durchführung von radiochirurgischen Eingriffen ermittelt werden, d. h. es kann beispielsweise der Bereich eines zu bestrahlenden Gewebes und/oder die Strahlendosis ermittelt werden, welche auf einen Ort einwirken soll, an welchem sich möglicherweise kleine Metastasen gebildet haben oder möglicherweise bilden werden, welche mit bisher bekannten Verfahren noch nicht aufgefunden werden können.

Weiterhin ist es möglich die erfindungsgemäß gewonnenen Informationen bezüglich möglicher Migrationswege und/oder gefährdeter Bereiche des Körpers zur Planung, Vorbereitung und/oder Durchführung einer Chemotherapie zu verwenden, d. h. es können beispielsweise Stellen bestimmt werden, an welchen zum Beispiel eine Infusion oder Injektion durchgeführt werden soll, wobei auch Informationen zum Typ und/oder zur Konzentration eines einzusetzenden Mittels, wie beispielsweise eines Chemotherapeutikums, ermittelt werden können. Bezüglich einer Vorrichtung und einem Verfahren zur Verabreichung einer Substanz, welche in Verbindung mit dem erfindungsgemäßen Verfahren und der nachfolgend beschriebenen erfindungsgemäßen Vorrichtung verwendet werden können, wird auf die am 30. November 2001 eingereichte europäische Patentanmeldung mit der Anmeldenummer 01 128 614.3 der Anmelderin verwiesen, wobei die in der dortigen Anmeldung enthaltene technische Lehre betreffend eine Vorrichtung und ein Verfahren zur Verabreichung einer Substanz in diese Anmeldung mit einbezogen werden. Ebenso können die für einen Körper ermittelten Migrationswege zur Bestimmung von Einbringpunkten von Zellen, wie zum Beispiel neuronalen Stammzellen oder modifizierten Zellen, verwendet werden, welche beispielsweise in einem oder mehreren gefährdeten Bereichen zur Prävention oder Behandlung einer möglichen Erkrankung dienen können.

Des Weiteren ist es möglich Daten zur Planung, Vorbereitung und/oder Unterstützung eines oder mehrerer chirurgischer Eingriffe unter Verwendung der erfindungsgemäß ermittelten möglichen Migrationswege von Zellen und/oder der ermittelten gefährdeten Bereiche des Körpers zu gewinnen, um einen möglichst frühzeitigen Eingriff bei möglicherweise von einer Erkrankung betroffenen Körper- oder Gewebebereichen durchführen zu können. Beispielsweise können bekannte Navigationsverfahren für gezielte chirurgische Eingriffe verwendet werden.

Allgemein können die erfindungsgemäß ermittelten Informationen zu möglichen Migrationswegen von Zellen oder Substanzen oder zu möglicherweise gefährdeten Bereichen eines Körpers oder Patienten vorteilhaft für eine lokale Behandlung von genau definierten Bereichen verwendet werden, da bei kleinen Bereichen zum Beispiel stark toxische Medikamente bei einer sogenannten lokalen Chemotherapie hochwirksam zum Beispiel unter Verwendung der in der europäischen Patentanmeldung Nr. 01 128 614.3 beschriebenen Vorrichtung eingesetzt werden können. Eine bei lokalen Bereichen mögliche hochwirksame relativ hohe Dosierung von toxischen Substanzen kann bei einer bekannten unspezifischen und nicht auf einen lokalen Bereich beschränkten Chemotherapie nicht eingesetzt werden. Ebenso können bei einer Behandlung durch Bestrahlung relativ hohe Strahlungsdosen verwendet werden, wenn nur ein relativ kleiner Gewebebereich bestrahlt wird, von welchem bekannt ist oder angenommen wird, dass sich dort möglicherweise erkranktes Gewebe, wie zum Beispiel Metastasen, bilden können oder schon gebildet haben. Eine unspezifische vorsorgliche Bestrahlung von großen Gewebebereichen mit hoher Strahlungsdosis verbietet sich demgegenüber aufgrund der Schädigung von gesundem Gewebe, welche in keinem Verhältnis zu einem möglichen Behandlungserfolg stehen würde. Erfindungsgemäß können somit durch die Kenntnis von möglichen Migrationswegen von Tumorzellen genau spezifizierte und häufig relativ kleine Gewebebereiche frühzeitig behandelt werden, so dass es durch die erfindungsgemäße Lehre möglich wird, das Wachstum zum Beispiel eines Tumors vorherzusagen, zu verfolgen und möglichst frühzeitig schon geeignete Gegenmaßnahmen zu ergreifen.

Vorteilhaft kann das erfindungsgemäße Verfahren zum Bestimmen von Migrationswegen von Zellen iterativ durchgeführt werden, d. h. es können zum Beispiel in einem ersten Schritt mögliche Migrationswege von Zellen ausgehend von einem oder mehreren Punkten oder Bereichen im Gewebe oder gefährdete Bereich des Körpers erfindungsgemäß ermittelt werden, wobei nachfolgend oder ergänzend Überprüfungs- oder Verifikationsverfahren durchgeführt werden können, bei welchen beispielsweise Testsubstanzen an bestimmten Stellen in einen Körper oder in Gewebe eingebracht und das Ausbreitungsverhalten dieser Substanzen, insbesondere die Migration dieser Substanzen und das Vorhandensein an bestimmten vorhergesagten Bereichen gemessen wird, um basierend darauf eine Präzisierung der erfindungsgemäß bestimmten Migrationswege durchzuführen. Weiterhin können zum Beispiel an den erfindungsgemäß ermittelten gefährdeten Bereichen gezielt Untersuchungen durchgeführt werden, um zum Beispiel Metastasen schon im Frühstadium erkennen zu können.

Die Erfindung bezieht sich weiterhin auf ein Computerprogramm, welches in den Speicher eines Computers geladen werden kann und Softwarecodeabschnitte umfasst, mit welchen einzelne oder mehrere der vorher beschriebenen Verfahrensschritte ausgeführt werden können, wenn das Programm auf einem Computer läuft. Weiterhin bezieht sich die Erfindung auf ein Computerprogrammprodukt, das auf einem computergeeignetem Medium oder Datenträger gespeichert ist und das vorher erwähnte Computerprogramm umfasst.

Gemäß einem weiteren Aspekt bezieht sich die vorliegende Erfindung auf eine Vorrichtung zum Bestimmen von Migrationswegen von Zellen, insbesondere von Tumorzellen mit einer Vorrichtung zum Erfassen von spezifischen Daten betreffend den Körper oder das Gewebe einer zu untersuchenden Person, insbesondere zur Durchführung eines bildgebenden Verfahrens, wobei eine Auswerteeinheit, zum Beispiel ein Computersystem vorgesehen ist zum erfindungsgemäßen Bestimmen von Migrationswegen. Vorteilhaft ist das Computersystem so ausgelegt, dass einer oder mehrere der oben beschriebenen Verfahrensschritte vollautomatisch und/oder interaktiv ausgeführt werden können. Dabei können herkömmliche Computer und/oder neuronale Netzwerke und/oder Fuzzy-Logik verwendet werden, um aus den gemessenen Daten erfindungsgemäß zum Beispiel mögliche Migrationswege von Zellen in einem Körper oder Gewebe zu ermitteln, wobei vorteilhaft auch Wahrscheinlichkeiten dafür ermittelt werden, dass sich Zellen über einen bestimmten der ermittelten Migrationswege ausbreiten und so beispielsweise Metastasen in gefährdeten Bereichen bilden. Erfindungsgemäß kann also beispielsweise ermittelt werden, an welchem Ort oder in welchem Gewebebereich Metastasen mit einer bestimmten Wahrscheinlichkeit auftreten, so dass geeignete Gegenmaßnahmen, wie zum Beispiel das Einbringen von Substanzen oder Zellen, eine Bestrahlung oder ein chirurgischer Eingriff präventiv oder zur Behandlung eingeleitet werden können. Dabei ist es erfindungsgemäß auch möglich für eine Person individuelle Maßnahmen vorzuschlagen und entsprechende Parameter für diese Maßnahmen, wie beispielsweise den Ort eines zu bestrahlenden Bereichs, die Strahlendosis, eine Infusions- oder Injektionsstelle, die Dosis einer einzubringenden Substanz oder weitere Parameter automatisch zu ermitteln und auszugeben.

Die erfindungsgemäße Vorrichtung kann weiterhin auch unterstützend für diagnostische, therapeutische oder chirurgische Verfahren betreffend die gefährdeten Bereiche verwendet werden.

Die Erfindung wird nachfolgend anhand von bevorzugten Ausführungsformen beschrieben werden. Es zeigen:
- Figur 1: ein Ablaufdiagramm einer Ausführungsform des erfindungsgemäßen Verfahrens;
- Figur 2: ein Ablaufdiagramm einer erweiterten Ausführungsform des erfindungsgemäßen Verfahrens; und
- Figur 3: eine schematische Darstellung einer erfindungsgemäßen Vorrichtung.

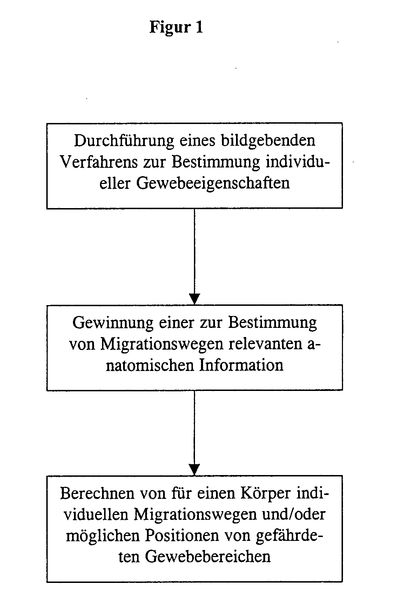

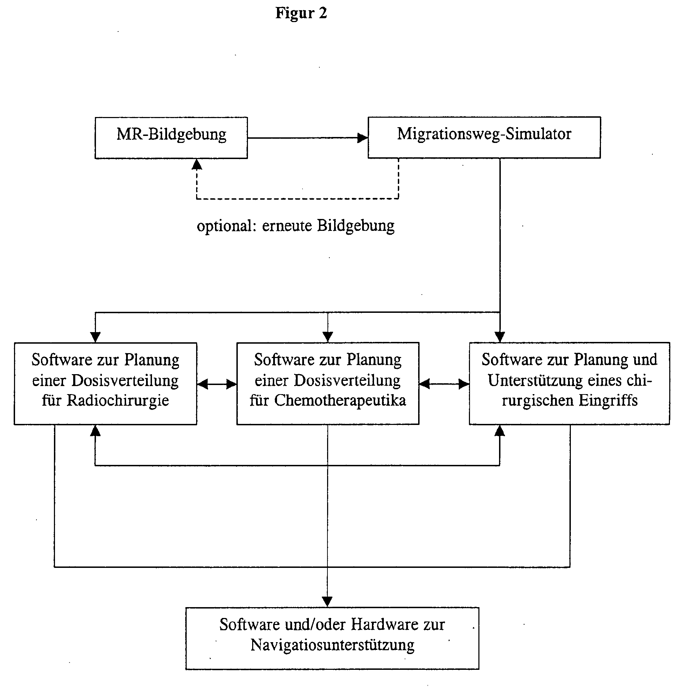

Figur 1 zeigt ein Ablaufdiagramm eines Verfahrens zum Bestimmen von Migrationswegen von Tumorzellen, wobei in einem ersten Schritt körper- oder personenspezifisch Gewebeeigenschaften durch bildgebende Verfahren ermittelt werden. Dabei können bekannte Vorrichtungen und Verfahren eingesetzt werden, welche zum Beispiel die Anordnung von Gewebe und Körperstrukturen erfassen können. Aus den so bestimmten Gewebeeigenschaften werden anatomische Informationen gewonnen, welche relevant für die Bestimmung von Migrationswegen von Zellen sind, wie zum Beispiel die Art des Gewebes, der Verlauf von Blutbahnen oder Diffusions- und Perfusionsparameter. Zur Bestimmung der Migrationswege relevante anatomische Informationen können kombiniert werden, um zum Beispiel den Migrationsweg von Zellen beginnend von einem oder mehreren Ausgangspunkten durch ein Gewebe mit bekannten Migrationseigenschaften und an das Gewebe angrenzenden Blutgefäßen mit bekannten Perfusionseigenschaften zu ermitteln und um so einen bestimmten, mehrere oder alle möglichen Migrationswege von Zellen aus einem bestimmten Bereich in verschiedene andere Bereiche entlang verschiedener Bahnen eines Körpers oder Gewebes zu ermitteln. Somit können schließlich verschiedene Migrationswege und mögliche oder wahrscheinliche Positionen von Tumorzellen oder Metastasen in gefährdeten Bereichen ermittelt werden, was eine frühzeitige und individuell abgestimmte Behandlung der Metastasen im Frühstadium ermöglicht, wie nachfolgend unter Bezugnahme auf Figur 2 beschrieben werden wird.

Figur 2 zeigt eine beispielhafte Ergänzung des in Figur 1 schematisch beschriebenen erfindungsgemäßen Verfahrens. Die von einem bildgebenden Verfahren, wie zum Beispiel MRI, erhaltenen Informationen werden in einen Simulator zum Ermitteln von möglichen Migrationswegen eingegeben, wobei basierend auf den vom Migrationsweg-Simulator ermittelten Daten optional weitere Daten durch ein bildgebendes Verfahren erfasst werden können, um zum Beispiel zu überprüfen, ob an den von dem Migrationsweg-Simulator ermittelten Positionen oder Gewebebereichen möglicherweise schon erkennbare Metastasen vorliegen. Basierend auf den so gewonnenen Ausgangsdaten können verschiedene Behandlungsarten einzeln oder in Kombination ausgewählt werden, wobei die Art einer durchzuführenden Behandlung und die für die Behandlung spezifischen Parameter bevorzugt automatisch zum Beispiel durch eine Software erzeugt werden können. Eine erste Behandlungsmöglichkeit ist die Planung und Vorbereitung eines radiochirurgischen Verfahrens, wobei eine Bestrahlungsvorrichtung unter Verwendung von bekannten Navigationsverfahren so angesteuert werden kann, dass die erfindungsgemäß ermittelten Metastasen bevorzugt bei eng umgrenzten definierten Gewebebereichen mit einer von einer Software ermittelten optimalen Dosisverteilung bestrahlt werden.

Eine weitere Behandlungsmöglichkeit ist die Planung einer Dosisverteilung für Chemotherapeutika, wobei beispielsweise ein oder mehrere vorteilhafte Orte zum Einbringen von Chemotherapeutika und die entsprechende Dosis bestimmt werden können, um die erfindungsgemäß aufgefundenen Metastasen zu behandeln.

Weiterhin ist es möglich unterstützende Informationen zum Beispiel für chirurgische Eingriffe zur Verfügung zu stellen. Dies können zum Beispiel Navigationsdaten sein, durch welche ein chirurgischer Eingriff zu einem gefährdeten Bereich mit Metastasen geführt werden kann.

Die beschriebenen optionalen Behandlungsverfahren können einzeln oder in Kombination durchgeführt werden, wobei jeweils eine möglichst vorteilhafte Behandlungsart, optional in Kombination mit einem oder mehreren weiteren Behandlungsverfahren ausgewählt werden kann, um zum Beispiel Gewebebereiche zu behandeln, bei welchen sich möglicherweise oder wahrscheinlich durch das erfindungsgemäße Verfahren oder die erfindungsgemäße Vorrichtung ermitteltes gefährdetes oder erkranktes Gewebe, wie zum Beispiel Metastasen befinden.

Allgemein können auch Maßnahmen ergriffen werden, um erkannte mögliche Migrationswege zu unterbrechen oder eine Ausbreitung von Zellen einzuschränken oder zu begrenzen. Hierzu können zum Beispiel präventiv chirurgische oder radiochirurgische Eingriffe oder eine Behandlung durch entsprechende Medikamente, wie zum Beispiel Chemotherapeutika durchgeführt werden.

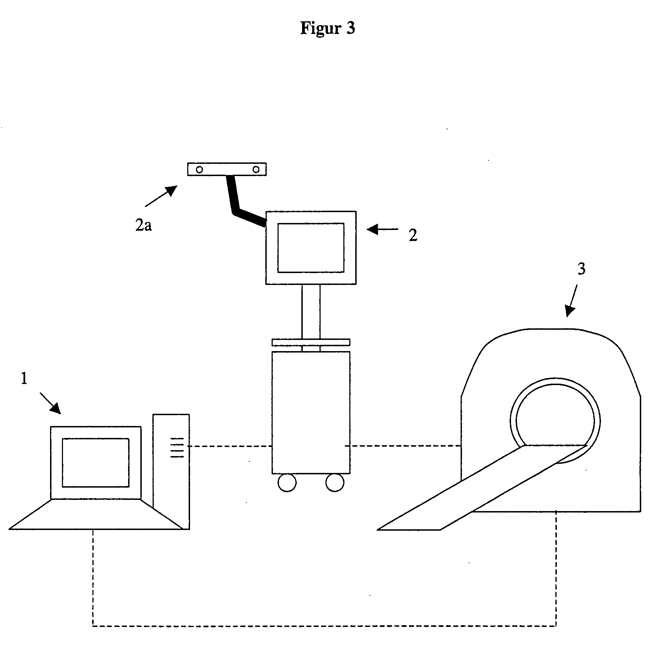

Figur 3 zeigt beispielhaft eine Ausführungsform einer erfindungsgemäßen Vorrichtung zum Bestimmen von Migrationswegen. Das in Figur 3 schematisch gezeigte VektorVision® -System weist ein Kernspinresonanz-System 3 auf, mit welchem Gewebeeigenschaften eines Körpers bestimmt werden können. Diese Daten werden an das Simulations-System 1 übertragen, um aus diesen Daten anatomische Informationen zu extrahieren, welche zur Bestimmung von Migrationswegen relevant sind. Die vom Simulations-Systems 1 ermittelten Daten möglicher Migrationswege von Zellen können zur Ansteuerung des Navigationssystems 2 verwendet werden, um zum Beispiel unter Verwendung der IR-Kameras 2a einen navigierten chirurgischen Eingriff bei einem Gewebebereich vornehmen zu können, bei welchem sich wahrscheinlich oder möglicherweise aufgrund der erfindungsgemäß ermittelten Migrationswege Metastasen bilden.

## Patentansprüche

1. Verfahren zum Bestimmen von Migrationswegen von Zellen, wobei Daten eines Körpers durch ein bildgebendes Verfahren erfasst werden, aus den erfassten Daten für Migrationswege relevante Informationen gewonnen werden, und individuell für den Körper Migrationswege berechnet werden.

2. Verfahren nach Anspruch 1, wobei als bildgebende Verfahren Kernspinresonanz-, Ultraschall-, Computertomographie-, Röntgen-, SPECT- oder PET-Verfahren verwendet werden.

3. Verfahren nach einem der vorhergehenden Ansprüche, wobei die für einen Körper berechneten Migrationswege zur Bestimmung der Lage von gefährdeten Bereichen verwendet werden.

4. Verfahren nach einem der vorhergehenden Ansprüche, wobei Diffusions-und/oder Perfusionsdaten zur Bestimmung von Migrationswegen in einem Körper verwendet werden.

5. Verfahren nach einem der vorhergehenden Ansprüche, wobei die für einen Körper ermittelten Migrationswege zur Bestimmung mindestens eines Einbringpunktes von Zellen in den Körper verwendet werden.

6. Verfahren nach einem der vorhergehenden Ansprüche, wobei die ermittelten Migrationswege und/oder Informationen über gefährdete Gewebebereiche zur Planung und Vorbereitung einer Bestrahlung, insbesondere zur Einstellung von Bestrahlungsparametern verwendet werden.

7. Verfahren nach einem der vorhergehenden Ansprüche, wobei die ermittelten Migrationswege und/oder Informationen über gefährdete Gewebebereiche zur Planung und Vorbereitung von chemotherapeutischen Verfahren verwendet werden.

8. Verfahren nach einem der vorhergehenden Ansprüche, wobei die ermittelten Migrationswege und/oder Informationen über gefährdete Gewebebereiche zur Planung und Vorbereitung von chirurgischen Eingriffen verwendet werden.

9. Verfahren nach einem der vorhergehenden Ansprüche, wobei das bildgebende Verfahren unter Verwendung der ermittelten Migrationswege und/oder der ermittelten gefährdeten Gewebebereiche durchgeführt wird.

10. Computerprogramm, welches in den Speicher eines Computers geladen werden kann und Softwarecodeabschnitte umfasst, mit welchen die Schritte gemäß einem der vorhergehenden Ansprüche ausgeführt werden können, wenn das Programm auf einem Computer läuft.

11. Computerprogrammprodukt, das auf einem computergeeigneten Medium oder Datenträger gespeichert ist und das Computerprogramm nach dem vorhergehenden Anspruch umfasst.

12. Vorrichtung zur Bestimmung von Migrationswegen von Zellen in einem Körper mit einer Vorrichtung (3) zur Untersuchung des Körpers, insbesondere zur Durchführung eines bildgebenden Verfahrens und einer Auswerteeinheit (1) zur Ermittlung von Migrationswegen in dem Körper basierend auf den von der Vorrichtung (3) zur Verfügung gestellten Daten.

13. Vorrichtung nach dem vorhergehenden Anspruch, wobei die Vorrichtung eine Recheneinheit zur Durchführung mindestens eines der in den Ansprüchen 1 bis 8 beschriebenen Verfahrensschritte enthält.
